# EUROPEAN PATENT SPECIFICATION

(11) **EP 1 599 089 B1**
(45) Date of publication and mention of the grant of the patent: **12.10.2011**
(21) Application number: 04712447.4
(22) Date of filing: 19.02.2004
(51) Int. Cl.: C12N 15/82, A01H 1/00

(54) **Efficient gene silencing in plants using short dsRNA sequences**
Effiziente Genabschaltung in Pflanzen unter Verwendung von kurzen dsRNA-Sequenzen
Blocage genique efficace chez des vegetaux; aide de courtes sequences dsRNA

(30) Priority: 19.02.2003 US 447711 P
(43) Date of publication of application: 30.11.2005
(73) Proprietor: Commonwealth Scientific and Industrial Research Organisation, Campbell, ACT 2612 (AU)
(72) Inventor: WANG, Ming, Bo, Kaleen, Australian Capital Territory 261 (AU); HELLIWELL, Christopher, Andrew, O'connor, Australian Capital Territory 2 (AU); WATERHOUSE, Peter, Michael, O'connor, Australian Capital Territory 2 (AU)
(74) Representative: Almond-Martin, Carol
(86) International application number: PCT/AU2004/000199
(87) International publication number: WO 2004/073390

(56) References cited:
- WO-A-02/059294
- WO-A1-98/53083
- WO-A1-99/53050
- WO-A2-00/63397
- YUKAWA YASUSHI ET AL: "Plant 7SL RNA and tRNA(Tyr) genes with inserted antisense sequences are efficiently expressed in an in vitro transcription system from Nicotiana tabacum cells." PLANT MOLECULAR BIOLOGY. NOV 2002, vol. 50, no. 4-5, November 2002 (2002-11), pages 713-723, XP002372879 ISSN: 0167-4412
- MARSHALLSAY C ET AL: "CHARACTERIZATION OF THE U3 AND U6 SNRNA GENES FROM WHEAT: U3 SNRNA GENES IN MONOCOT PLANTS ARE TRANSCRIBED BY RNA POLYMERASE III" PLANT MOLECULAR BIOLOGY, SPRINGER, DORDRECHT, NL, vol. 19, no. 6, 1992, pages 973-983, XP001037859 ISSN: 0167-4412
- WATERHOUSE PETER M ET AL: "Exploring plant genomes by RNA-induced gene silencing." NATURE REVIEWS GENETICS, vol. 4, no. 1, January 2003 (2003-01), pages 29-38, XP002372880 ISSN: 1471-0056
- WANG MING-BO ET AL: "Application of gene silencing in plants." CURRENT OPINION IN PLANT BIOLOGY. APR 2002, vol. 5, no. 2, April 2002 (2002-04), pages 146-150, XP002372881 ISSN: 1369-5266
- WATERHOUSE PM ET AL: 'Virus resistance and gene silencing in plants can be induced by simultaneous expression of sense and antisiense RNA' PROC NATL ACAD SCI USA vol. 95, no. 23, November 1998, pages 13959 - 13964, XP002114472
- KLAHRE U ET AL: 'High molecular weight RNAs and small interfering RNAs induce systematic posttransscriptional gene silencing in plants' PROC NATL ACAD SCI USA vol. 99, no. 18, 03 September 2002, pages 11981 - 11986, XP002248496
- PADDISON PJ ET AL: 'Short hairpin rnas (shrnas) induce sequence-specific silencing in mammalian cells' GENES & DEVELOPMENT vol. 16, no. 8, 15 April 2002, pages 948 - 958, XP002204653
- MCMANUS MT ET AL: 'Gene silencing using micro - RNA designed hairpins' RNA vol. 8, no. 6, June 2002, pages 842 - 850, XP002296480
- GENE THERAPY SYSTEMS INC: 'GeneSilencer sh RNA vectors' CATALOG, [Online] 2002, Retrieved from the Internet: <URL:http://www.genetherapysystems.com/cata log/index.cfm>
- AMBION INC.: 'RNA interference and gene silencing - history and overvieuw', [Online] 2002, XP002290958 Retrieved from the Internet: <URL:http://www.ambion.com/techlib/hottopic s/rnai_may2002_print.html>
- WANG MING-BO ET AL: "Hairpin RNAs derived from RNA polymerase II and polymerase III promoter-directed transgenes are processed differently in plants", RNA (COLD SPRING HARBOR), vol. 14, no. 5, May 2008 (2008-05), pages 903-913, ISSN: 1355-8382
- YUKAWA YASUSHI ET AL: "Plant 7SL RNA genes belong to type 4 of RNA polymerase III- dependent genes that are composed of mixed promoters.", THE PLANT JOURNAL : FOR CELL AND MOLECULAR BIOLOGY JUL 2005 LNKD- PUBMED:15960619, vol. 43, no. 1, July 2005 (2005-07), pages 97-106, ISSN: 0960-7412

### SEQUENCE LISTING

<110> Commonwealth Scientific and Industrial Reseach Organization
<120> Efficient gene silencing in plants using short dsRNA sequences
<130> BCS-03-2001
<150> US 60/447,711
   <151> 2003-02-19
<160> 33
<170> PatentIn version 3.1
<210> 1
   <211> 341
   <212> DNA
   <213> Artificial Sequence
<220>
   <223> sequence of the promoter of the 7SL-2 gene of Arabidopsis thalian
   a var. Landsberg erecta
<220>
   <221> misc_feature
   <222> (1)..(6)
   <223> XhoI restriction site
<220>
   <221> misc_feature
   <222> (7)..(322)
   <223> PolIII promoter region
<220>
   <221> misc_feature
   <222> (336)..(341)
   <223> XhoI restriction site
<220>
   <221> misc_feature
   <222> (329)..(335)
   <223> poly T nucleotide stretch
<220>
   <221> misc_feature
   <222> (323)..(328)
   <223> SalI restriction site
<400> 1
   ctcgagatgt tgttgttacc agaaagtaaa taaatgttca atctctgatg ttctcaagta     60
   agtgagtttt attgggaata atattaactc atgttcttct gcatttgatt cctttgccgc    120
   tctcttcttc tatcttaaat ctgtgtatac tatttcacta ttgggctttt tattagtcta    180
   taatgggact caaaataagg ctttggccca catcaaaaag ataagtcaca aatcaaaact    240
   aaattcagag tcttttctcc cacatcggtc actgtactca ttttgtgttt gtttatatat    300
   tacacgaacc gatctttgtt acgtcgactt tttttctcga g                         341
<210> 2
   <211> 429
   <212> DNA
   <213> Artificial Sequence
<220>
   <223> sequence of the promoter of the 7SL-2 gene of Arabidopsis thalian
   a var. Landsberg erecta including 86 bases downstream of the tran
   scription initiation site.
<220>
   <221> misc_feature
   <222> (1)..(6)
   <223> XhoI restriction site
<220>
   <221> misc_feature
   <222> (424)..(429)
   <223> XhoI restriction site
<220>
   <221> misc_feature
   <222> (415)..(423)
   <223> poly T stretch
<220>
   <221> misc_feature
   <222> (409)..(414)
   <223> SalI restriction site
<220>
   <221> misc_feature
   <222> (7)..(408)
   <223> PolIII promoter region.
<400> 2
   ctcgagatgt tgttgttacc agaaagtaaa taaatgttca atctctgatg ttctcaagta     60
   agtgagtttt attgggaata atattaactt atgttcttct tgcatttgat ttctttgccg    120
   ctctcttctt ctatcttaaa tctgtgtata ctatttcact attgggcttt ttattagtct    180
   ataatgggac tcaaaataag gctttggccc acatcaaaaa gataagtcac aaatcaaaac    240
   taaattcaga gtcttttctc ccacatcggt cactgtactc ttttgtgttt gtttatatat    300
   tacacgaacc gatctttggt acgtcgagct aagtaacatg agcttgtaac ccatgtgggg    360
   acattaagat ggtggaacac tggctcgggt ccacgggccg gttctgttgt cgactttttt    420
   tttctcgag                                                           429
<210> 3
   <211> 334
   <212> DNA
   <213> Artificial Sequence
<220>
   <223> sequence of the promoter of the U3 snRNA of Arabidopsis thaliana
   var. Landsberg erecta
<220>
   <221> misc_feature
   <222> (1)..(6)
   <223> EcoRI restriction site
<220>
   <221> misc_feature
   <222> (314)..(319)
   <223> PvuI restriction site
<220>
   <221> misc_feature
   <222> (320)..(328)
<223> poly T stretch
<220>
   <221> misc_feature
   <222> (329)..(334)
   <223> EcoRI restriction site
<220>
   <221> misc_feature
   <222> (7)..(313)
   <223> Pol III promoter region
<400> 3
   gaattcttat gcagcctgtg atggataact gaatcaaaca aatggcgtct gggtttaaga     60
   agatctgttt tggctatgtt ggacgaaaca agtgaacttt taggatcaac ttcagtttat    120
   atatggagct tatatcgagc aataagataa gtgggctttt tatgtaattt aatgggctat    180
   cgtccataga ttcactaata cccatgccca gtacccatgt atgcgtttca tataagctcc    240
   taatttctcc cacatcgctc aaatctaaac aaatcttgtt gtatatataa cactgaggga    300
   gcaacattgg tcacgatcgt ttttttttga attc                                334
<210> 4
   <211> 467
   <212> DNA
   <213> Artificial Sequence
<220>
   <223> sequence of the promoter of the U3 snRNA gene of Arabidopsis thal iana var. Landsberg erecta including 136 bases downstream of the transcription initiation site.
<220>
   <221> misc_feature
   <222> (1)..(6)
   <223> EcoRI restriction site
<220>
   <221> misc_feature
   <222> (7)..(446)
   <223> Pol III promoter region
<220>
   <221> misc_feature
   <222> (447)..(452)
   <223> XhoI restriction site
<220>
   <221> misc_feature
   <222> (453)..(461)
   <223> poly T stretch
<220>
   <221> misc_feature
   <222> (462)..(467)
   <223> EcoRI restriction site
<400> 4
   gaattcttat gcagcctgtg atggataact gaatcaaaca aatggcgtct gggtttaaga     60
   agatctgttt tggctatgtt ggacgaaaca agtgaacttt taggatcaac ttcagtttat    120
   atatggagct tatatcgagc aataagataa gtgggctttt tatgtaattt aatgggctat    180
   cgtccataga ttcactaata cccatgccca gtacccatgt atgcgtttca tataagctcc    240
   taatttctcc cacatcgctc aaatctaaac aaatcttgtt gtatatataa cactgaggga    300
   gcaacattgg tcacgacctt acttgaacag gatctgttct ataggctcgt acctctgttt    360
   ccttgatttc tcaagagaca ggcccttaac cctggttgat gaaccatgac cgtgcggcta    420
   gagcgtgatt gacggctacg atcgtcctcg agtttttttt tgaattc                  467
<210> 5
   <211> 456
   <212> DNA
   <213> Artificial Sequence
<220>
   <223> sequence of the promoter of the U6 snRNA gene of Arabidopsis thal iana var. Landsberg erecta including 3 bases downstream of the tr anscription initiation site
<220>
   <221> misc_feature
   <222> (1)..(6)
   <223> XhoI restriction site
<220>
   <221> misc_feature
   <222> (7)..(436)
   <223> Pol III promoter region
<220>
   <221> misc_feature
   <222> (437)..(442)
   <223> SalI restriction site
<220>
   <221> misc_feature
   <222> (443)..(450)
   <223> poly T stretch
<220>
   <221> misc_feature
   <222> (451)..(456)
   <223> Sac I restriction site
<400> 5
   ctcgagcttc gttgaacaac ggaaactcga cttgccttcc gcacaataca tcatttcttc     60
   ttagcttttt ttcttcttct tcgttcatac agtttttttt tgtttatcag cttacatttt    120
   cttgaaccgt agctttcgtt ttcttctttt taactttcca ttcggagttt ttgtatcttg    180
   tttcatagtt tgtcccagga ttagaatgat taggcatcga accttcaaga atttgattga    240
   ataaaacatc ttcattctta agatatgaag ataatcttca aaaggcccct gggaatctga    300
   aagaagagaa gcaggcccat ttatatggga aagaacaata gtatttctta tataggccca    360
   tttaagttga aaacaatctt caaaagtccc acatcgctta gataagaaaa cgaagctgag    420
   tttatataca gctagagtcg actttttttt gagctc                              456
<210> 6
   <211> 488
   <212> DNA
   <213> Artificial Sequence
<220>
   <223> sequence of the promoter of the U3 snRNA gene of Arabidopsis thal iana var. Landsberg erecta including 20 bases downstream of the t ranscription initiation site
<220>
   <221> misc_feature
   <222> (1)..(6)
   <223> XhoI restriction site
<220>
   <221> misc_feature
   <222> (7)..(468)
   <223> Pol III promoter region
<220>
   <221> misc_feature
   <222> (469)..(474)
   <223> PvuI restriction site
<220>
   <221> misc_feature
   <222> (475)..(482)
   <223> Poly T stretch
<220>
   <221> misc_feature
   <222> (483)..(488)
   <223> XhoI restriction site
<400> 6
   ctcgagcttc gttgaacaac ggaaactcga cttgccttcc gcacaataca tcatttcttc     60
   ttagcttttt ttcttcttct tcgttcatac agtttttttt tgtttatcag cttacatttt    120
   cttgaaccgt agctttcgtt ttcttctttt taactttcca ttcggagttt ttgtatcttg    180
   tttcatagtt tgtcccagga ttagaatgat taggcatcga accttcaaga atttgattga    240
   ataaaacatc ttcattctta agatatgaag ataatcttca aaaggcccct gggaatctga    300
   aagaagagaa gcaggcccat ttatatggga aagaacaata gtatttctta tataggccca    360
   tttaagttga aaacaatctt caaaagtccc acatcgctta gataagaaaa cgaagctgag    420
   tttatataca gctagagtcg aagtagtgat tgtcccttcg gggacatccg atcgtttttt    480
   ttctcgag                                                             488
<210> 7
   <211> 405
   <212> DNA
   <213> Artificial Sequence
<220>
   <223> sequence of the promoter of the U3 snRNA of rice (Oryza sativa In
   dica IR36)
<220>
   <221> misc_feature
   <222> (1)..(6)
   <223> EcoRI restriction site
<220>
   <221> misc_feature
   <222> (7)..(384)
   <223> Pol III promoter region
<220>
   <221> misc_feature
   <222> (385)..(390)
   <223> PvuI restriction site
<220>
   <221> misc_feature
   <222> (391)..(399)
   <223> poly T stretch
<220>
   <221> misc_feature
   <222> (400)..(405)
   <223> EcoRI restriction site
<400> 7
   gaattcaagg gatctttaaa catacgaaca gatcacttaa agttcttctg aagcaactta      60
   aagttatcag gcatgcatgg atcttggagg aatcagatgt gcagtcaggg accatagcac     120
   aggacaggcg tcttctactg gtgctaccag caaatgctgg aagccgggaa cactgggtac     180
   gttggaaacc acgtgatgtg gagtaagata aactgtagga gaaaagcatt tcgtagtggg     240
   ccatgaagcc tttcaggaca tgtattgcag tatgggccgg cccattacgc aattggacga     300
   caacaaagac tagtattagt accacctcgg ctatccacat agatcaaagc tggtttaaaa     360
   gagttgtgca gatgatccgt ggcacgatcg tttttttttg aattc                     405
<210> 8
   <211> 442
   <212> DNA
   <213> Artificial Sequence
<220>
   <223> sequence of the promoter of the U3 snRNA of tomato (a garden vari
   ety with small gourd-shaped yellow fruit)
<220>
   <221> misc_feature
   <222> (1)..(6)
   <223> EcoRI restriction site
<220>
   <221> misc_feature
   <222> (7)..(421)
   <223> Pol III promoter region
<220>
   <221> misc_feature
   <222> (422)..(427)
   <223> PvuI restriction site
<220>
   <221> misc_feature
   <222> (428)..(436)
   <223> Poly T stretch
<220>
   <221> misc_feature
   <222> (437)..(442)
   <223> EcoRI restriction site
<400> 8
   gaattctgag agcattgtgt ggcgttcctc tgaattactt actgtcactt tgattggagc      60
   cattattttc agactctact gaagattgaa ttgaatgaga aactatgaaa ctttacaagt     120
   gaattattat ggagttcatg gcaactgcta tggagttttt cctactggga attggaacgg     180
   tttctacgaa attaactgtc cacacgttaa aaatataaat taatgcgtaa ttgttatttt     240
   ttctataaca aataaaaaac tgaaatacga cataaatttt attactttaa ttgcacttta     300
   gccttagaga tattgcgttg tagtcggcgt aggtgtgtca ggggccaata tattgttccc     360
   acatcggcag tgcagcacat aaactctagc gttataagaa tctatccact atcaacggtc     420
   acgatcgttt ttttttgaat tc                                              442
<210> 9
   <211> 295
   <212> DNA
   <213> Artificial Sequence
<220>
   <223> sequence of the dsRNA encoding region of 94bp for silencing expre
   ssion of the GUS gene (GUShp94)
<220>
   <221> misc_feature
   <222> (1)..(6)
   <223> SalI restriction site
<220>
   <221> misc_feature
   <222> (6)..(11)
   <223> PvuI restriction site
<220>
   <221> misc_feature
   <222> (12)..(100)
   <223> GUS sequence (sense)
<220>
   <221> misc_feature
   <222> (101)..(195)
   <223> spacer sequence
<220>
   <221> misc_feature
   <222> (190)..(195)
   <223> BamHI restriction site
<220>
   <221> misc_feature
   <222> (196)..(284)
   <223> GUS sequence (antisense)
<220>
   <221> misc_feature
   <222> (285)..(290)
   <223> PvuI restriction site
<220>
   <221> misc_feature
   <222> (290)..(295)
   <223> SalI restriction site
<400> 9
   gtcgacgatc gcagcgtaat gctctacacc acgccgaaca cctgggtgga cgatatcacc     60
   gtggtgacgc atgtcgcgca agactgtaac cacgcgtctg ttgactggca ggtggtggcc    120
   aatggtgatg tcagcgttga actgcgtgat gcggatcaac aggtggttgc aactggacaa    180
   ggcactagcg ggatccagac gcgtggttac agtcttgcgc gacatgcgtc accacggtga    240
   tatcgtccac ccaggtgttc ggcgtggtgt agagcattac gctgcgatcg tcgac         295
<210> 10
   <211> 93
   <212> DNA
   <213> Artificial Sequence
<220>
   <223> sequence of the dsRNA encoding region of 41 bp for silencing expr
   ession of the GUS gene (GUShp41)
<220>
   <221> misc_feature
   <222> (1)..(6)
   <223> SalI restriction site
<220>
   <221> misc_feature
   <222> (7)..(42)
   <223> GUS sequence (sense)
<220>
   <221> misc_feature
   <222> (43)..(51)
   <223> spacer sequence
<220>
   <221> misc_feature
   <222> (52)..(87)
   <223> GUS sequence (antisense)
<220>
   <221> misc_feature
   <222> (88)..(93)
   <223> Sal I restriction site
<400> 10
   gtcgactggg cagatgaaca tggcatcgtg gtgattgatg aatgcgagaa cttcatcaat     60
   caccacgatg ccatgttcat ctgcccagtc gac                                 93
<210> 11
   <211> 50
   <212> DNA
   <213> Artificial Sequence
<220>
   <223> sequence of the dsRNA encoding region of 21 bp for silencing expr
   ession of the GUS gene (GUShp21)
<220>
   <221> misc_feature
   <222> (1)..(6)
   <223> SalI restriction site
<220>
   <221> misc_feature
   <222> (7)..(22)
   <223> GUS sequence (sense)
<220>
   <221> misc_feature
   <222> (23)..(28)
   <223> spacer sequence
<220>
   <221> misc_feature
   <222> (29)..(44)
   <223> GUS sequence (antisense)
<220>
   <221> misc_feature
   <222> (45)..(50)
   <223> Sal I restriction site
<400> 11
   gtcgactggg cagatgaaca tgtacgatca tgttcatctg cccagtcgac 50
<210> 12
   <211> 94
   <212> DNA
   <213> Artificial Sequence
<220>
   <223> sequence of the dsRNA encoding region of 42 bp for silencing expr ession of the PHYB gene, derived from the 5' end of PHYB (PHYB5hp 42)-upper strand
<400> 12
   tcgacggagt cgggggtagt ggcggtggcc gtggcggtgg ccgtggagga ggccacggcc     60
   accgccacgg ccaccgccac tacccccgac tccg                                 94
<210> 13
   <211> 94
   <212> DNA
   <213> Artificial Sequence
<220>
   <223> sequence of the dsRNA encoding region of 42 bp for silencing expr ession of the PHYB gene, derived from the 5' end of PHYB (PHYB5hp 42)-lower strand
<400> 13
   tcgacggagt cgggggtagt ggcggtggcc gtggcggtgg ccgtggcctc ctccacggcc 60
   accgccacgg ccaccgccac tacccccgac tccg 94
<210> 14
   <211> 52
   <212> DNA
   <213> Artificial Sequence
<220>
   <223> sequence of the dsRNA encoding region of 21 bp for silencing expr ession of the PHYB gene, derived from the 5' end of PHYB (PHYB5hp 21)-upper strand
<400> 14
   tcgacggagt cgggggtagt ggcggaggag gccgccacta cccccgactc cg 52
<210> 15
   <211> 52
   <212> DNA
   <213> Artificial Sequence
<220>
   <223> sequence of the dsRNA encoding region of 21 bp for silencing expr ession of the PHYB gene, derived from the 5' end of PHYB (pHYB5hp 21)-lower strand
<400> 15
   tcgacggagt cgggggtagt ggcggcctcc tccgccacta cccccgactc cg 52
<210> 16
   <211> 94
   <212> DNA
   <213> Artificial Sequence
<220>
   <223> sequence of the dsRNA encoding region of 42 bp for silencing expr ession of the PHYB gene, derived from the center of PHYB (PHYBChp 42)-upper strand
<400> 16
   tcgatggatg gtgtggttca gccatgtagg gatatggcgg gggaacagga gggttccccc 60
   gccatatccc tacatggctg aaccacacca tcca 94
<210> 17
   <211> 94
   <212> DNA
   <213> Artificial Sequence
<220>
   <223> sequence of the dsRNA encoding region of 42 bp for silencing expr ession of the PHYB gene, derived from the center of PHYB (PHYBChp 42)-lower strand
<400> 17
   tcgatggatg gtgtggttca gccatgtagg gatatggcgg gggaaccctc ctgttccccc      60
   gccatatccc tacatggctg aaccacacca tcca                                  94
<210> 18
   <211> 52
   <212> DNA
   <213> Artificial Sequence
<220>
   <223> sequence of the dsRNA encoding region of 21 bp for silencing expr ession of the PHYB gene, derived from the center of PHYB (PHYBChp 21)-upper strand
<400> 18
   tcgatggatg gtgtggttca gccataggag gatggctgaa ccacacctcc aa 52
<210> 19
   <211> 52
   <212> DNA
   <213> Artificial Sequence
<220>
   <223> sequence of the dsRNA encoding region of 21 bp for silencing expr ession of the PHYB gene, derived from the center of PHYB (PHYBChp 21)-lower strand
<400> 19
   tcgatggatg gtgtggttca gccatcctcc tatggctgaa ccacaccatc ca 52
<210> 20
   <211> 94
   <212> DNA
   <213> Artificial Sequence
<220>
<223> sequence of the dsRNA encoding region of 42 bp for silencing expr ession of the PHYB gene, derived from the 3' end of PHYB (PHYB3hp 42)-upper strand
<400> 20
   tcgacattgt caactgctag tggaagtggt gacatgatgc tgatgaagga ggtcatcagc      60
   atcatgtcac cacttccact agcagttgac aatg                                  94
<210> 21
   <211> 94
   <212> DNA
   <213> Artificial Sequence
<220>
   <223> sequence of the dsRNA encoding region of 42 bp for silencing expr ession of the PHYB gene, derived from the 3' end of PHYB (PHYB3hp 42)-lower strand
<400> 21
   tcgacattgt caactgctag tggaagtggt gacatgatgc tgatgacctc cttcatcagc      60
   atcatgtcac cacttccact agcagttgac aatg                                  94
<210> 22
   <211> 52
   <212> DNA
   <213> Artificial Sequence
<220>
   <223> sequence of the dsRNA encoding region of 21 bp for silencing expr ession of the PHYB gene, derived from the 3' end of PHYB (PHYB3hp 21)-upper strand
<400> 22
   tcgacattgt caactgctag tggaaaggag gttccactag cagttgacaa tg 52
<210> 23
   <211> 52
   <212> DNA
   <213> Artificial Sequence
<220>
   <223> sequence of the dsRNA encoding region of 21 bp for silencing expr ession of the PHYB gene, derived from the 3' end of PHYB (PHYB3hp 21)-lower strand
<400> 23
   tcgacattgt caactgctag tggaacctcc tttccactag cagttgacaa tg 52
<210> 24
   <211> 94
   <212> DNA
   <213> Artificial Sequence
<220>
   <223> sequence of the dsRNA encoding region of 42 bp for silencing expr
   ession of the PDS gene (PDS42)-upper strand
<400> 24
   tcgacttaac ttgtaaggaa tattacgatc ctaaccggtc aatgctagga ggagcattga 60
   ccggttagga tcgtaatatt ccttacaagt taag 94
<210> 25
   <211> 94
   <212> DNA
   <213> Artificial Sequence
<220>
<223> sequence of the dsRNA encoding region of 42 bp for silencing expr
   ession of the PDS gene (PDS42)-lower strand
<400> 25
   tcgacttaac ttgtaaggaa tattacgatc ctaaccggtc aatgctcctc ctagcattga 60
   ccggttagga tcgtaatatt ccttacaagt taag 94
<210> 26
   <211> 52
   <212> DNA
   <213> Artificial Sequence
<220>
   <223> sequence of the dsRNA encoding region of 21 bp for silencing expr
   ession of the PDS gene (PDS21)-upper strand
<400> 26
   tcgacttaac ttgtaaggaa tattaaggag gtaatattcc ttacaagtta ag 52
<210> 27
   <211> 52
   <212> DNA
   <213> Artificial Sequence
<220>
   <223> sequence of the dsRNA encoding region of 21 bp for silencing expr
   ession of the PDS gene (PDS21)-lower strand
<400> 27
   tcgacttaac ttgtaaggaa tattacctcc ttaatattcc ttacaagtta ag 52
<210> 28
   <211> 115
   <212> DNA
   <213> Artificial sequence
<220>
   <223> small hairpin RNA coding region (GUS_A)
<220>
   <221> misc_feature
   <222> (1)..(11)
   <223> SalI/PvuI restriction sites
<220>
   <221> misc_feature
   <222> (12)..(53)
   <223> sense RNA encoding region
<220>
   <221> misc_feature
   <222> (54)..(62)
   <223> loop structure
<220>
   <221> misc_feature
   <222> (63)..(104)
   <223> antisense RNA encoding region
<220>
   <221> misc_feature
   <222> (105)..(115)
   <223> SalI/PvuI restriction sites
<400> 28
   gtcgacgatc gtgcggtcac tcattacggc aaagtgtggg tcaataatca ggagttcctt     60
   cttcctgatt attgacccac actttgccgt aatgagtgac cgcagtcgac gatcg         115
<210> 29
   <211> 112
   <212> DNA
   <213> Artificial sequence
<220>
   <223> small hairpin RNA coding region (GUS_B)
<220>
   <221> misc_feature
   <222> (1)..(8)
   <223> SalI/PvuI restriction sites
<220>
   <221> misc_feature
   <222> (9)..(50)
   <223> sense RNA encoding sequence
<220>
   <221> misc_feature
   <222> (51)..(59)
   <223> loop structure
<220>
   <221> misc_feature
   <222> (60)..(101)
   <223> antisense RNA coding region
<220>
   <221> misc_feature
   <222> (102)..(112)
   <223> SalI/pvuI restriction site
<400> 29
   gtcgacgatc gtcatgaaga tgcggacttg cgtggcaaag gattcgataa gttccttctt     60
   tatcgaatcc tttgccacgc aagtccgcat cttcatgacg agtcgacgat cg            112
<210> 30
   <211> 115
   <212> DNA
   <213> Artificial sequence
<220>
   <223> small hairpin RNA coding region (GUS_C)
<220>
   <221> misc_feature
   <222> (1)..(11)
   <223> SalI/PvuI restriction sites
<220>
   <221> misc_feature
   <222> (12)..(53)
   <223> sense RNA coding region
<220>
   <221> misc_feature
   <222> (54)..(62)
   <223> loop structure
<220>
<221> misc_feature
   <222> (63)..(104)
   <223> antisense RNA encoding region
<220>
   <221> misc_feature
   <222> (105)..(115)
   <223> SalI/PvuI restriction sites
<400> 30
   gtcgacgatc gtgcgacctc gcaaggcata ttgcgcgttg gcggtaacaa gaagttcctt     60
   ctttcttgtt accgccaacg cgcaatatgc cttgcgaggt cgcagtcgac gatcg         115
<210> 31
   <211> 115
   <212> DNA
   <213> Artificial sequence
<220>
   <223> small hairpin RNA coding region (EIN_A)
<220>
   <221> misc_feature
   <222> (1)..(11)
   <223> SalI/PvuI restriction sites
<220>
   <221> misc_feature
   <222> (12)..(53)
   <223> sense RNA encoding region
<220>
   <221> misc_feature
   <222> (54)..(62)
   <223> loop structure
<220>
   <221> misc_feature
   <222> (63)..(104)
   <223> antisense RNA encoding region
<220>
   <221> misc_feature
   <222> (105)..(115)
   <223> SalI/PvuI restriction sites
<400> 31
   gtcgacgatc gcatcttatg ccaatatgtt gcagctcgca taagcgttgt gacgttcctt     60
   ctgtcacaac gcttatgcga gctgcaacat attggcataa gatggtcgac gatcg         115
<210> 32
   <211> 112
   <212> DNA
   <213> Artificial sequence
<220>
   <223> small hairpin RNA coding region (EIN_B)
<220>
   <221> misc_feature
   <222> (1)..(8)
<223> SalI/PvuI restriction sites
<220>
   <221> misc_feature
   <222> (9)..(50)
   <223> sense RNA coding region
<220>
   <221> misc_feature
   <222> (51)..(59)
   <223> loop structure
<220>
   <221> misc_feature
   <222> (60)..(101)
   <223> antisense RNA coding region
<220>
   <221> misc_feature
   <222> (102)..(112)
   <223> SalI/PvuI restriction site
<400> 32
   gtcgacgatc ggcaggcctg gtattacttc tctatgtttc tggcgtcttg gttccttctc     60
   aagacgccag aaacatagag aagtaatacc aggcctgccg agtcgacgat cg            112
<210> 33
   <211> 115
   <212> DNA
   <213> Artificial sequence
<220>
   <223> small hairpin RNA coding region (EIN_C)
<220>
   <221> misc_feature
   <222> (1)..(11)
   <223> SalI/PvuI restriction site
<220>
   <221> misc_feature
   <222> (12)..(53)
   <223> sense RNA encoding region
<220>
   <221> misc_feature
   <222> (54)..(62)
   <223> loop structure
<220>
   <221> misc_feature
   <222> (63)..(104)
   <223> antisense RNA encoding region
<220>
   <221> misc_feature
   <222> (105)..(115)
   <223> SalI/PvuI restriction site
<400> 33
   gtcgacgatc gcatagctgt ttcctgtgtg aaattggtat ccgctcacaa ttcgttcctt     60
   ctgaattgtg agcggatacc aatttcacac aggaaacagc tatggtcgac gatcg         115

## Description

### FIELD OF THE INVENTION

The current invention relates generally to the field of genetic modification of plants, more particularly to the use of short double stranded (dsRNA) sequences to deliberately silence the expression of one or more genes in plant cells and plants. Methods and means are provided to increase the efficiency of gene silencing when using dsRNA sequences which have a stem length shorther than about 200 basepairs.

### BACKGROUND

The mechanism of posttranscriptional silencing of gene expression in plants and animals triggered by target-gene specific dsRNA, provided either exogenously or endogenously through transcription of dsRNA encoding chimeric genes, has recently become the subject of numerous studies. Since the initial description of this phenomenon in animals and plants (Fire et al., 1998; Hamilton et al., 1998 ; Waterhouse et al., 1998 ), it has become clear that the dsRNA is processed by an RNAse with a preference for dsRNA (such as DICER in Drosophila) into short, approximately 21 nucleotide long RNA molecules that are used as guide sequences, providing sequence-specificity to a complex capable of degrading specific mRNA molecules.

The high specificity and efficiency of gene silencing initiated by dsRNA that is homologous to the gene to be silenced rapidly turned this methodology into the preferred tool to generate eukaryotic organisms wherein expression of one or more specific transcribed nucleotide sequences is reduced or inactivated. Such reduction or inactivation of the expression of a gene of interest may be achieved with a goal to produce eukaryotic organisms with a preferred phenotype (see e.g. WO 02/029028, wherein Brassica plants are generated which develop sepals instead petals using dsRNA technology). Reduction or inactivation of expression of transcribed sequences also plays an important role in experimental studies trying to allocate a function to the wealth of nucleotide sequences which have become available through various genome sequencing programs. Particularly for the latter, it may be advantageous to use short dsRNA sequences, since such oligonucleotides may conveniently be generated *in vitro.* In higher animals, the use of short dsRNA molecules is preferred in view of the fact that larger dsRNA molecules seem to trigger interferon responses (Elbashir et al. 2001).

Up to now, the production of inhibitory RNA (used herein to describe antisense RNA, sense RNA and dsRNA) inside the cells of eukaryotic organisms, mostly occurs through the action of DNA dependent RNA polymerase II (PolII) recognizing the common PolII type promoters.

Antisense RNA production through the action of RNA polymerase III in plants has been documented.

Bourque and Folk (1992) described suppression of the expression of a CAT gene, transiently delivered to plant cells, by co-electroporation with a DNA comprising inverted sequences of the chloramphenicol actetyltransferase reporter gene, fused to a soybean tRNA^{met} gene lacking a terminator, such that the tRNA^{met} sequences caused the transcription of CAT antisense sequences by RNA polymerase III.

US 5,354,854 describes an expression system and method to use the same in plants to suppress gene expression, the system including a constitutive promoter element from a tRNA gene and an antisense strand DNA fused to the promoter element for being co-transcribed with the promoter element by an RNA polymerase III to suppress expression of a gene.

Yukawa et al. 2002 described antisense RNA sequences targeted against conserved structural elements or domains in the RNAs of potato spindle tuber viroid, hop latent viroid and potato virus S which were embedded in the anticodon region or a Nicotiana tRNAtyr gene or near the 3' end of an Arabidopsis 7SL RNA gene, and demonstrated in vitro transcription of such chimeric genes in a homologous plant extract.

EP 0 387 775 describes and claims a DNA molecule, optionally occuring in multiple copies, containing sections of a gene transcribed by polymerase III and a DNA sequence encoding for an inhibiting RNA molecule, characterized in that it contains the transcription units of a tRNA gene necessary for transcription by polymerase III, including the sequence which determine the secondary structure of the tRNA, and that the DNA sequence coding for the inhibiting RNA molecule is arranged inside the DNA molecule in such a way that the inhibiting RNA molecule is a part of the transcript.

Expression of small interfering RNAs in mammalian cells has recently been well documented. Paddison et al. 2002 ; Sook Lee et al. 2002 ; Miyagishi et al. 2002, Sui et al. 2002 ; Brummelkamp et al, 2002 and Paul et al. 2002, all describe the expression of small interfering RNA in human or mammalian cells using RNA polymerase III specific promoters derived from either H1-RNA or U6 snRNA.

US 6,146,886 describes and claims a transcribed non-naturally occuring RNA molecule comprising a desired RNA portion, wherein said non-naturally occuring RNA molecule comprises an intramolecular stem formed by base-pairing interactions between a 3' region and 5' complementary nucleotides in said RNA, wherein said intramolecular stem comprises at least 8 basepairs ; wherein said desired RNA portion is selected from the group consisting of antisense RNA, decoy RNA, enzymatic RNA, agonist RNA and antagonist RNA, wherin said RNA molecule is transcribed by a type 2 RNA polymerase III promoter system.

The prior art remains however deficient in providing methods for highly efficient expression of small interfering dsRNAs in plant cells. This problem has been solved as hereinafter described.

### SUMMARY OF THE INVENTION

The invention provides methods for reducing the expression of a gene of interest in a plant cell, comprising the following steps :
(a) providing a chimeric gene to the plant cell, the chimeric gene comprising the following operably linked DNA fragments :
   i) a promoter recognized by a DNA dependent RNA polymerase will of the plant cell characterized in that the promoter is a promoter of type III (type 3) preferably a type 3 POLIII promoter selected from the promoter of a gene encoding U6snRNA , the promoter of a gene encoding U3snRNA, , more preferably a promoter comprising the nucleotide sequence of promoter is selected from the nucleotide sequences of SEQ ID Nr 3, SEQ ID Nr 4, SEQ ID Nr 5, SEQ ID Nr 6, SEQ ID Nr 7 or SEQ ID Nr 8;
   ii) a DNA fragment which, when transcribed, yields an RNA molecule, the RNA molecule comprising a sense and antisense nucleotide sequence,
      (1) the sense nucleotide sequence comprising about 19 contiguous nucleotides having about 90 to about 100% sequence identity to a nucleotide sequence of about 19 contiguous nucleotide sequences from the RNA transcribed from the gene of interest;
      (2) the antisense nucleotide sequence comprising about 19 contiguous nucleotides having about 90 to 100% sequence identity to the complement of a nucleotide sequence of about 19 contiguous nucleotide sequence of the sense sequence; wherein the sense and antisense nucleotide sequence are capable of forming a double stranded RNA of about 19 to about 200 nucleotides in length ; and
   iii) an oligo dT stretch comprising at least 4 consecutive T-residues; wherein said chimeric gene is characterized in that said chimeric gene has all its cis-acting elements which interact with said DNA dependent RNA polymerase III upstream of the region transcribed by RNA polymerase III; and
(b) identifying plant cells wherein the expression of the gene of interest is reduced when compared to the expression of the gene of interest in plant cells which do not comprise the chimeric gene.

The invention further provides a chimeric gene comprising the following operably linked DNA fragments :
i) a promoter recognized by a DNA dependent RNA polymerase III of the plant cell characterized in that the promoter is a promoter of type III , preferably a type 3 POLIII promoters selected from the promoter of a gene encoding U6snRNA , the promoter of a gene encoding U3snRNA, the more preferably a promoter comprising the nucleotide sequence of promoter is selected from the nucleotide sequences of SEQ ID Nr 3, SEQ ID Nr 4, SEQ ID Nr 5, SEQ ID Nr 6, SEQ ID Nr 7 or SEQ ID Nr 8;
ii) a DNA fragment which, when transcribed, yields an RNA molecule, the RNA molecule comprising a sense and antisense nucleotide sequence,
   (1) the sense nucleotide sequence comprising about 19 contiguous nucleotides having about 90 to about 100% sequence identity to a nucleotide sequence of about 19 contiguous nucleotide sequences from the RNA transcribed from a gene of interest in a plant cell;
   (2) the antisense nucleotide sequence comprising about 19 contiguous nucleotides having about 90 to 100% sequence identity to the complement of a nucleotide sequence of about 19 contiguous nucleotide sequence of the sense sequence; wherein the sense and antisense nucleotide sequence are capable of forming a double stranded RNA of about 19 to about 200 nucleotides in length ;
   wherein said chimeric gene is characterized in that said chimeric gene has all its cis-acting elements which interact with said DNA dependent RNA polymerase III upstream of the region transcribed by RNA polymerase III; and
iii) an oligo dT stretch comprising at least 4 consecutive T-residues.

The invention further provides plant cell and plants comprising the above mentioned chimeric genes

### BRIEF DESCRIPTION OF THE FIGURE

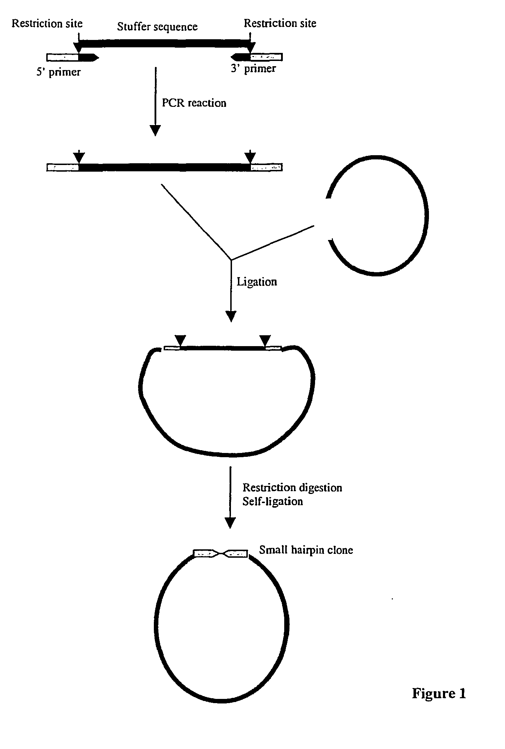

Figure 1 outlines schematically a convenient cloning strategy for creating and handling a coding region encoding short dsRNA sequences.

### DETAILED DESCRIPTION

The current invention is based on the observation that chimeric genes encoding short dsRNA molecules, preferably ranging between about 20 basepairs (bp) and about 100 bp under control of type 3 promoters recognized by RNA polymerase III, resulted in more efficient gene silencing than similar constructs driven by the strong constitutive RNA Polymerase II promoter CaMV 35S.

These type 3 promoters have the additional advantage that all required cis-acting elements of the promoter are located in the region upstream of the transcribed region, in contrast to type 2 promoters recognized by RNA polymerase III, which had been used in the prior art to direct expression of antisense RNA.

Thus, in a first embodiment, the current invention relates to a method for reducing the expression of a gene of interest in a plant cell, comprising the following steps :
(a) providing a chimeric gene to the plant cell, the chimeric gene comprising the following operably linked DNA fragments :
   i) a promoter recognized by a DNA dependent RNA polymerase III of the plant cell whereby the promoter is a promoter of type 3
   ii) a DNA fragment which, when transcribed, yields an RNA molecule, the RNA molecule comprising a sense and antisense nucleotide sequence, and wherein
      (1) the sense nucleotide sequence comprises about 19 contiguous nucleotides having about 90 to about 100% sequence identity to a nucleotide sequence of about 19 contiguous nucleotide sequences from the RNA transcribed from the gene of interest;
      (2) the antisense nucleotide sequence comprising about 19 contiguous nucleotides having about 90 to 100% sequence identity to the complement of a nucleotide sequence of about 19 contiguous nucleotide sequence of the sense sequence; wherein the sense and antisense nucleotide sequence are capable of forming a double stranded RNA of about 19 to about 200 nucleotides in length ; and
   iii) an oligo dT stretch comprising at least 4 consecutive T-residues; wherein said chimeric gene is characterized in that said chimeric gene has all its cis-acting elements which interact with said DNA dependent RNA polymerase III upstream of the region transcribed by RNA polymerase III; and
(b) identifying plant cells wherein the expression of the gene of interest is reduced when compared to the expression of the gene of interest in plant cells which do not comprise the chimeric gene.

As used herein « a promoter recognized by the DNA dependent RNA polymerase III » is a promoter which directs transcription of the associated DNA region through the polymerase action of RNA polymerase III. These include genes encoding 5S RNA, tRNA, U6 snRNA and a few other small stable RNAs, many involved in RNA processing. Most of the promoters used by Pol III require sequence elements downstream of +1, within the transcribed region. A minority of pol III templates however, lack any requirement for intragenic promoter elements. These are referred to as type 3 promoters. In other words, « type 3 Pol III promoters », are those promoters which are recognized by RNA polymerase III and contain all cis-acting elements, interacting with the RNA polymerase III upstream of the region normally transcribed by RNA polymerase III. Such type 3 Pol III promoters can thus easily be combined in a chimeric gene with a heterologous region, the transcription of which is desired, such as the dsRNA coding regions of the current invention.

Typically, type 3 Pol III promoters contain a TATA box (located between -25 and -30 in Human U6 snRNA gene) and a Proximal Sequence element (PSE ; located between -47 and - 66 in Human U6 snRNA). They may also contain a Distal Sequence Element (DSE ; located between -214 and -244 in Human U6 snRNA).

Type 3 Pol III promoters can be found e.g. associated with the genes , U3 snRNA and U6 snRNA. Such sequences have been isolated from Arabidopsis, rice and tomato and representative sequences of such promoters are represented in the sequence listing under the entries SEQ ID No 3-8.

Other nucleotide sequences for type 3 Pol III promoters can be found in nucleotide sequence databases under the entries for the *Humulus lupulus* H17SL-1 gene (AJ236706), *Humulus lupulus* H17SL-2 gene (AJ236704), *Humulus lupulus* H17SL-3 gene (AJ236705), *Humulus lupulus* H17SL-4 gene (AJ236703), *A. thaliana* U6-1 snRNA gene (X52527), *A. thaliana* U6-26 snRNA gene (X52528), *A. thaliana* U6-29 snRNA gene (X52529), *A. thaliana* U6-1 snRNA gene (X52527), *Zea mays* U3 snRNA gene (Z29641), *Solanum tuberosum* U6 snRNA gene (Z17301; X 60506; S83742), Tomato U6 smal nuclear RNA gene (X51447), *A. thaliana* U3C snRNA gene (X52630), A. thaliana U3B snRNA gene (X52629), *Oryza sativa* U3 snRNA promoter (X79685), Tomato U3 smal nuclear RNA gene (X14411), *Triticum aestivum* U3 snRNA gene (X63065), *Triticum aestivum* U6 snRNA gene (X63066).

It goes without saying that variant type 3 Pol III promoters may be isolated from other varieties of tomato, rice or Arabidopsis, or from other plant species without little experimentation. E.g. libraries of genomic clones from such plants may be isolated using U6 snRNA or U3 snRNA coding sequences (such as the coding sequences of any of the above mentioned sequences identified by their accession number and additionally the *Vicia faba* U6snRNA coding sequence (X04788), or the maize DNA for U6 snRNA (X52315) as a probe, and the upstream sequences, preferably the about 300 to 400 bp upstream of the transcribed regions may be isolated and used as type 3 Pol III promoters. Alternatively, PCR based techniques such as inverse-PCR or TAIL^{®}-PCR may be used to isolate the genomic sequences including the promoter sequences adjacent to known transcribed regions. Moreover, any of the type 3 PolIII promoter sequences attached or of the above mentioned promoter sequences, identified by their accession numbers, may be used as probes under stringent hybridization conditions or as source of information to generate PCR primers to isolate the corresponding promoter sequences from other varieties or plant species.

"Stringent hybridization conditions" as used herein mean that hybridization will generally occur if there is at least 95% and preferably at least 97% sequence identity between the probe and the target sequence. Examples of stringent hybridization conditions are overnight incubation in a solution comprising 50% formamide, 5 x SSC (150 mM NaCl, 15 mM trisodium citrate), 50 mM sodium phosphate (pH 7.6), 5x Denhardt's solution, 10% dextran sulfate, and 20 µg/ml denatured, sheared carrier DNA such as salmon sperm DNA, followed by washing the hybridization support in 0.1 x SSC at approximately 65 °C. Other hybridization and wash conditions are well known and are exemplified in Sambrook et al, Molecular Cloning: A Laboratory Manual, Second Edition, Cold Spring Harbor, NY (1989), particularly chapter 11.

Although the type 3 Pol III promoters have no requirement for cis-acting elements located with the transcribed region, it is clear that sequences normally located downstream of the transcription initiation site may nevertheless be included in the chimeric constructs of the invention.

It has also been observed that type 3 Pol III promoters originally isolated from monocotyeldonous plants can be used to good effect in both dicotyledonous and monocotyleodous plant cells and plants, whereas type 3 Pol III promoters originally isolated from dicotyledonous plants can only be efficiently used in dicotyledonous plant cells and plants. Moreover, the most efficient gene silencing has been obtained when chimeric genes were used comprising a type 3 Pol III promoter derived from the same or closely related species.

As used herein, a « gene of interest » may be any nucleic acid of interest, which is transcribed (or replicated) into an RNA molecule, and which is prone to post transcriptional RNA degradation. These include but are not limited to transgenes, endogenous genes and transcribed viral sequences. It will also be immediately apparent that for the methods of the invention, it is not required to have knowledge of the nucleotide sequence of the gene of interest. Indeed, it may be possible to directly derive small fragments and operably link them in inverted repeat orientation, under control of a type 3 Pol III promoter

As indicated above, the transcribed DNA region should be capable of encoding an RNA molecule comprising a sense and antisense nucleotide region, whereby the sense nucleotide sequence comprises about 19 contiguous nucleotides having about 90 to about 100% sequence identity to a nucleotide sequence of about 19 contiguous nucleotide sequences from the RNA transcribed from the gene of interest and whereby the antisense nucleotide sequence comprising about 19 contiguous nucleotides having about 90 to 100% sequence identity to the complement of a nucleotide sequence of about 19 contiguous nucleotide sequence of the sense sequence. The sense and antisense nucleotide sequence should be capable of forming a double stranded RNA of about 19 to about 200 nucleotides, particularly about 21 to about 90 or 100 nucleotides, more particularly about 40 to about 50 nucleotides in length. However, the length of the dsRNA stem may also be about 30, about 60, about 70 or about 80 nucleotides in length. It will be clear that where the dsRNA region is larger than 19 nucleotides, there is only a requirement that there is at least one double stranded region of about 19 nucleotides (whereby there can be about one mismatch between the sense and antisense region) the sense strand of which is « identical » (allowing for one mismatch) with 19 consecutive nucleotides of the target nucleic acid or gene of interest.

For the purpose of this invention, the "sequence identity" of two related nucleotide sequences, expressed as a percentage, refers to the number of positions in the two optimally aligned sequences which have identical residues (x100) divided by the number of positions compared. A gap, i.e. a position in an alignment where a residue is present in one sequence but not in the other is regarded as a position with non-identical residues. The alignment of the two sequences is performed by the Needleman and Wunsch algorithm (Needleman and Wunsch 1970) Computer-assisted sequence alignment, can be conveniently performed using standard software program such as GAP which is part of the Wisconsin Package Version 10.1 (Genetics Computer Group, Madison, Wisconsin, USA) using the default scoring matrix with a gap creation penalty of 50 and a gap extension penalty of 3.

The transcribed DNA region may comprise a stretch of nucleotides ranging from 3 to about 100 nucleotides or more specifically from about 6 to about 40 nucleotides, which are located between the sense and antisense encoding nucleotide region, and which are not related to the nucleotide sequence of the target gene ( a so-called spacer region).

The chimeric genes of the current invention, comprising a transcribed DNA region with short antisense and sense fragments may conveniently be constructed using a stuffer DNA sequence between the short antisense and sense fragments during the cloning procedures, which may thereafter be removed. To that end, the stuffer segment may be equipped with restriction enzymes recognitions sites, such as rare-cutting restriction enzymes for the easy removal of the stuffer sequence and re-ligation (self-ligation) of the cloning vector, whereby the short sense and antisense region are now brought in vicinity of each other. As outlined in Figure 1, a DNA fragment comprising a short sense sequence, a short, antisense sequence complementary to the sense sequence, and a stuffer DNA sequence may be conveniently construct by PCR amplification using oligonucleotide primers comprising the sense or antisense sequence and a sequence corresponding to part of the stuffer DNA sequence.

The above mentioned « oligo dT stretch » is a stretch of consecutive T-residues which serve as a terminator for the RNA polymerase III activity. It should comprise at least 4 T-residues, but obviously may contain more T-residues.

Chimeric genes according to the invention may be provided to plant cells by introduction into plant cells using any means of DNA transformation available in the art, including but not limited to Agrobacterium mediated transformation, microprojectile bombardment, direct DNA uptake into protoplasts or plant tissues (by electroporation, PEG-mediated uptake, etc.) and may result in transiently or stably transformed plant cells. The chimeric genes may also be provided to the plant cells using viral vectors, capable of replicating in plant cells. Chimeric genes may also be provided to plant cells by crossing parental plants, at least one of which comprises a chimeric gene according to the invention.

As used herein, « reducing the expression of a gene of interest » refers to the comparison of the expression of the gene of interest in the plant cell in the presence of the dsRNA or chimeric genes of the invention, to the expression of the gene of interest in the absence of the dsRNA or chimeric genes of the invention. The expression in the presence of the chimeric RNA of the invention should thus be lower than the expression in absence thereof, e.g. be only about 75% or 50% or 10% or about 5% of the expression in absence of the chimeric RNA. The expression may be completely inhibited for all practical purposes by the presence of the chimeric RNA or the chimeric gene encoding such an RNA.

A reduction of expression of a gene of interest may be measured as a reduction in transcription of (part of) that gene, a reduction in translation of (part of) that gene or a reduction in the effect the presence of the transcribed RNA(s) or translated polypeptide(s) have on the plant cell or the plant, and will ultimately lead to altered phenotypic traits. It is clear that the reduction in expression of a gene of interest, may be accompanied by or correlated to an increase in expression of another gene. Although the main effect of dsRNA is the post-transcriptional degradation of specific RNA, effects of dsRNA on the transcription process have been documented. Such additional effects will also contribute to the reduction of expression of a gene of interest mediated by dsRNA.

Other embodiments of the invention relate to the chimeric genes as herein described, as well as to plants, plant cells, plant tissues or seeds comprising the chimeric genes of the invention.

It is also an object of the invention to provide plant cells and plants containing the chimeric genes according to the invention. Gametes, seeds, embryos, either zygotic or somatic, progeny or hybrids of plants comprising the chimeric genes of the present invention, which are produced by traditional breeding methods are also included within the scope of the present invention.

The methods and means described herein are believed to be suitable for all plant cells and plants, both dicotyledonous and monocotyledonous plant cells and plants including but not limited to cotton, Brassica vegetables, oilseed rape, wheat, com or maize, barley, sunflowers, rice, oats, sugarcane, soybean, vegetables (including chicory, lettuce, tomato), tobacco, potato, sugarbeet, papaya, pineapple, mango, Arabidopsis thaliana, but also plants used in horticulture, floriculture or forestry.

The following non-limiting Examples describe the construction of chimeric genes for the reduction of the expression of a gene of interest in a plant cell by small dsRNA and the use of such genes.

Unless stated otherwise in the Examples, all recombinant DNA techniques are carried out according to standard protocols as described in Sambrook et al. (1989) Molecular Cloning: A Laboratory Manual, Second Edition, Cold Spring Harbor Laboratory Press, NY and in Volumes 1 and 2 of Ausubel et al. (1994) Current Protocols in Molecular Biology, Current Protocols, USA. Standard materials and methods for plant molecular work are described in Plant Molecular Biology Labfax (1993) by R.D.D. Croy, jointly published by BIOS Scientific Publications Ltd (UK) and Blackwell Scientific Publications, UK. Other references for standard molecular biology techniques include Sambrook and Russell (2001) Molecular Cloning: A Laboratory Manual, Third Edition, Cold Spring Harbor Laboratory Press, NY, Volumes I and II of Brown (1998) Molecular Biology LabFax, Second Edition, Academic Press (UK). Standard materials and methods for polymerase chain reactions can be found in Dieffenbach and Dveksler (1995) PCR Primer: A Laboratory Manual, Cold Spring Harbor Laboratory Press, and in McPherson at al. (2000) PCR - Basics: From Background to Bench, First Edition, Springer Verlag, Germany.

Throughout the description and Examples, reference is made to the following sequences:
- SEQ ID No. 1:: sequence of the promoter of the 7SL-2 gene of Arabidopsis thaliana var. Landsberg erecta, followed by a unique restriction site in front of an oligo dT stretch (for reference only).
- SEQ ID No. 2:: sequence of the promoter of the 7SL-2 gene of Arabidopsis thaliana var. Landsberg erecta including 86 bases downstream of the transcription
- SEQ ID No. 3:: initiation site, followed by a unique restriction site in front of an oligo dT stretch (for reference only). sequence of the promoter of the U3B snRNA of Arabidopsis thaliana var. Landsberg erecta, followed by a unique restriction site in front of an oligo dT stretch.
- SEQ ID No. 4:: sequence of the promoter of the U3B snRNA gene of Arabidopsis thaliana var. Landsberg erecta including 136 bases downstream of the transcription initiation site, followed by a unique restriction site in front of an oligo dT stretch.
- SEQ ID No. 5:: sequence of the promoter of the U6-26 snRNA gene of Arabidopsis thaliana var. Landsberg erecta including 3 bases downstream of the transcription initiation site, followed by a unique restriction site in front of an oligo dT stretch.
- SEQ ID No. 6:: sequence of the promoter of the U6-26 snRNA gene of Arabidopsis thaliana var. Landsberg erecta including 20 bases downstream of the transcription initiation site, followed by a unique restriction site in front of an oligo dT stretch.
- SEQ ID No. 7:: sequence of the promoter of the U3 snRNA of rice (Oryza sativa Indica IR36), followed by a unique restriction site in front of an oligo dT stretch.
- SEQ ID No. 8:: sequence of the promoter of the U3 snRNA of tomato (a garden variety with small gourd-shaped yellow fruit), followed by a unique restriction site in front of an oligo dT stretch.
- SEQ ID No. 9:: sequence of the dsRNA encoding region of 94bp for silencing expression of the GUS gene (GUShp94).
- SEQ ID No. 10:: sequence of the dsRNA encoding region of 41 bp for silencing expression of the GUS gene (GUShp41).
- SEQ ID No. 11:: sequence of the dsRNA encoding region of 21 bp for silencing expression of the GUS gene (GUShp21).
- SEQ ID No. 12:: sequence of the dsRNA encoding region of 42 bp for silencing expression of the PHYB gene, derived from the 5' end of PHYB (PHYB5hp42)-upper strand.
- SEQ ID No. 13:: sequence of the dsRNA encoding region of 42 bp for silencing expression of the PHYB gene, derived from the 5' end of PHYB (PHYB5hp42)-lower strand.
- SEQ ID No. 14:: sequence of the dsRNA encoding region of 21 bp for silencing expression of the PHYB gene, derived from the 5' end of PHYB (PHYB5hp21)-upper strand.
- SEQ ID No. 15:: sequence of the dsRNA encoding region of 21 bp for silencing expression of the PHYB gene, derived from the 5' end of PHYB (PHYB5hp21)-lower strand.
- SEQ ID No. 16:: sequence of the dsRNA encoding region of 42 bp for silencing expression of the PHYB gene, derived from the center of PHYB (PHYBChp42)-upper strand.
- SEQ ID No. 17:: sequence of the dsRNA encoding region of 42 bp for silencing expression of the PHYB gene, derived from the center of PHYB (PHYBChp42)-lower strand.
- SEQ ID No. 18:: sequence of the dsRNA encoding region of 21 bp for silencing expression of the PHYB gene, derived from the center of PHYB (PHYBChp21)-upper strand.
- SEQ ID No. 19:: sequence of the dsRNA encoding region of 21 bp for silencing expression of the PHYB gene, derived from the center of PHYB (PHYBChp21)-lower strand.
- SEQ ID No. 20:: sequence of the dsRNA encoding region of 42 bp for silencing expression of the PHYB gene, derived from the 3' end of PHYB (PHYB3hp42)-upper strand.
- SEQ ID No. 21:: sequence of the dsRNA encoding region of 42 bp for silencing expression of the PHYB gene, derived from the 3' end of PHYB (PHYB3hp42)-lower strand.
- SEQ ID No. 22:: sequence of the dsRNA encoding region of 21 bp for silencing expression of the PHYB gene, derived from the 3' end of PHYB (PHYB3hp21)-upper strand.
- SEQ ID No. 23:: sequence of the dsRNA encoding region of 21 bp for silencing expression of the PHYB gene, derived from the 3' end of PHYB (PHYB3hp21)-lower strand.
- SEQ ID No. 24:: sequence of the dsRNA encoding region of 42 bp for silencing expression of the PDS gene (PDS42)-upper strand.
- SEQ ID No. 25:: sequence of the dsRNA encoding region of 42 bp for silencing expression of the PDS gene (PDS42)-lower strand.
- SEQ ID No. 26:: sequence of the dsRNA encoding region of 21 bp for silencing expression of the PDS gene (PDS21)-upper strand.
- SEQ ID No. 27:: sequence of the dsRNA encoding region of 21 bp for silencing expression of the PDS gene (PDS21)-lower strand.
- SEQ ID No. 28:: sequence of a dsRNA encoding region of 42 bp for silencing expression of a GUS gene (GUS-A)
- SEQ ID No. 29:: sequence of a dsRNA encoding region of 42 bp for silencing expression of a GUS gene (GUS-B).
- SEQ ID No. 30:: sequence of a dsRNA encoding region of 42 bp for silencing expression of a GUS gene (GUS-C).
- SEQ ID No. 31:: sequence of a dsRNA encoding region of 42 bp for silencing expression of EIN (EIN-A).
- SEQ ID No. 32:: sequence of a dsRNA encoding region of 42 bp for silencing expression of EIN (EIN-B).
- SEQ ID No. 33:: sequence of a dsRNA encoding region of 42 bp for silencing expression of EIN (EIN-C).

### EXAMPLES

### Example 1. Construction of type 3 PoI III promoter -oligodT stretch cassettes.

Type 3 Pol III promoters and 7SL promoters (for reference only) were isolated from Arabidopsis, rice or tomato , U3snRNA or U6snRNA genes using PCR amplification, designed in such a way that
a) the resulting fragments were flanked by restriction enzyme recognition sites not present within the amplified fragment;
b) the promoter fragments were followed by a unique restriction site (SalI, XhoI or PvuI), followed by
c) a poly(T) sequence (with 7-9 T residues) as Pol III terminator.

In some of the cloned promoter fragments, additional sequences of the coding region downstream of the transcription initiation site were included to investigate the possible effect of conserved motifs in the coding region of the small RNAs on transcription and/or gene silencing. The resulting fragments (represented in SEQ IDs No 1 to 8) were cloned in intermediate cloning vectors (see Table 1). Sense, antisense or inverted repeat sequences can readily be inserted in the unique restriction site between the type 3 Pol III promoters and the polyT stretch.

**Table 1. Cloned PolIII promoter-terminator cassettes cloned***

| Small RNAs | Cloned promoters | Size (bp)*** | Plant species | Name of intermediate plasmid |
|---|---|---|---|---|
| 7SL-2 (for reference only) | At7SL-P At7SL+86** | 343 432 | Arabidopsis *(L.er)* | pMBW444 pMBW445 |
| U3B | AtU3B-P AtU3B+136 | 334 467 | Arabidopsis *(L.er)* | pMBW442 pMBW426 |
| U6-26 | AtU6+3 AtU6+20 | | Arabidopsis *(L.er)* | PWGEM.U6+3 PWGEM.U3+20 |
| U3 | OsU3-P | 407 | Rice (Oryza sativa indica IR36) | pMBW446-LW |
| U3 | TomU3-P | 443 | Tomato (a garden variety with small gourd-shaped yellow fruit) | pMBW443-LW |

| | | | | |
|---|---|---|---|---|
| **This number represents the sequence from the coding region of the small RNA gene. ***The sizes given include the restriction sites and the oligo (dT)s added to the PCR primers. | | | | |

### Example 2. Testing of the PolIII promoters in gene silencing constructs against a GUS reporter gene (Nicotiana tabacum).

To test these PolIII promoters for silencing, a GUS inverted-repeat sequence (SEQ ID No 9) was synthesized, which consists of 186 bp sense sequence of GUS (nt. 690-875 of GUS coding sequence) fused at the 3' end with an antisense version of the first 94 bp in the 186 bp fragment (nt. 690-783 of GUS coding sequence). This i/r sequence is flanked by two SalI sites and two PvuI sites, and can therefore be cloned into the PolIII promoter vectors as a SalI or PvuI fragment. Constructs were prepared with all the PolIII promoters described in Table 1 using the i/rGUS sequence (GUShp94) (see Table 2). In addition to the GUShp94 sequence, constructs were also prepared with the AtU3B+136 promoter (SEQ ID No 4.) and the CaMV35S promoter using smaller i/r GUS sequences such GUShp41a (41 bp in the stem spaced by a 9 bp non-GUS sequence ; SEQ ID No 10) and GUShp21 (21 bp in the stem spaced by a 6 bp non-GUS sequence, SEQ ID No 11) (Table 2).

**Table 2. Summary of constructs tested in tobacco**

| **Constructs** | **Description** |
|---|---|
| pMBW465 | GUShp94 driven by 35S promoter (pART7) |
| pMBW466 | GUShp94 driven by AtU3+136 |
| pMBW468 | GUShp94 driven by AtU3 |
| pMBW470 | GUShp94 driven by At7SL (for reference only) |
| pMBW472 | GUShp94 driven by At7SL+86 |
| pMBW473 | GUShp94 driven by OsU3 |
| pMBW476 | GUShp94 driven by AtU3+3 |
| pMBW477 | GUShp94 driven by AtU3+20 |
| pLMW64 | GUShp94 driven by TomU3 |
| pLMW53 | GUShp41a driven by 35S promoter |
| pLMW58 | GUShp41 driven by AtU3+136 |
| pLMW61 | GUShp21c driven by AtU3+136 |

These constructs were introduced to binary vectors pART27 or pWBVec4a for plant transformation. Two different transgenic tobacco lines expressing GUS, were transformed by all these constructs. A control construct in which the GUShp94 sequence was driven by a 35S promoter (in pART7) was also included.

Leaf tissue from transformed tobacco plantlets on rooting medium was assayed for GUS activity (fluorometric MUG assay) and the results are summarized in Table 3.

The results show that the GUShp94 constructs with AtU3 (pMBW468), At7SL (pMBW470) (for reference only), At7SL+86 (pMBW472) (for reference only), AtU3+3 (pMBW476), AtU3+20 (pMBW477) and TomU3 (pLMW64) promoters all activated silencing of the GUS gene in tobacco. The AtU3, AtU6+20, and TomU3 constructs appeared to perform better than the others. The AtU3+136 construct (pMBW466) did not seem to give significant GUS silencing in tobacco. Also, the OsU3 construct (pMBW473) appeared to confer only a low level of GUS silencing. The PolIII promoter construct pLMW58 (AtU3+136-GUShp41a) gave significant levels of GUS silencing in tobacco whereas the 35S construct pLMW53 (35S-GUShp41a) did not, suggesting that the PolIII promoters are more effective than the PolII promoters in driving the expression of small hairpin RNA.

**Table 3. MUG assay of tobacco leaf tissue transformed with constructs listed in Table 2 (5 µg protein)**

| Cons truct s | Untr ansform ed | 465 | 466 | 468 | 470 | 472 | 473 | 476 | 477 | 64 | 53 | 58 | 61 |
|---|---|---|---|---|---|---|---|---|---|---|---|---|---|
| PPG H2 GUS back grou nd | 40.0 | 2.0 | 17.4 | 2.2 | 8.2 | 5.6 | 11.3 | 8.4 | 5.2 | 7.9 | 55.4 | 20.6 | 6.0 |
| | 51.1 | 21.4 | 34.0 | 12.8 | 10.9 | 5.6 | 17.0 | 8.2 | 3.9 | 6.6 | 53.4 | 17.2 | 19.4 |
| | 51.0 | 1.8 | 13.9 | 4.5 | 9.2 | 12.8 | 14.9 | 22.9 | 35.5 | 2.9 | 27.0 | 1.4 | 25.8 |
| | 46.8 | 0.6 | 16.0 | 12.92 | 6.9 | 7.8 | 4.2 | 36.5 | 3.7 | 20.9 | | 9.1 | 29.6 |
| | | 0.7 | 24.7 | 6.9 | 12.5 | 9.9 | 15.3 | | 8.4 | 11.0 | | | 24.1 |
| | | 3.5 | 20.2 | 7.1 | | 10.8 | 2.1 | | 10.9 | 9.0 | | | 21.6 |
| | | 23.3 | | | | 19.1 | 17.1 | | 1.8 | 6.7 | | | 25.5 |
| | | | | | | 5.5 | 24.3 | | 32.7 | 3.3 | | | 13.7 |
| | | | | | | 13.3 | 20.5 | | 3.9 | 13.6 | | | 31.3 |
| | | | | | | 13.9 | 20.8 | | | | | | 57.4 |
| | | | | | | | | | | | | | 43.5 |
| | | | | | | | | | | | | | 12.8 |
| PPH G24 GUS back grou nd | 17.7 | 18.9 | 39.8 | 19.8 | 2.54 | 1.7 | 9.2 | 13.4 | 14.5 | 6.6 | 50.0 | 30.6 | 14.6 |
| | 32.4 | 0.5 | 23.9 | 18.1 | 38.8 | 20.6 | 11.0 | 18.2 | 9.1 | | 48.4 | 9 | 43.9 |
| | 54.6 | | 15.5 | 4.3 | 15.5 | 5.8 | 16.7 | 4.2 | | | 25.9 | | 26.8 |
| | 18.6 | | 13.8 | 9.3 | 8.2 | | 14.8 | 10.9 | | | | | 8.6 |
| | | | | 5.0 | | | 12.9 | | | | | | |
| | | | | 11.6 | | | 16.5 | | | | | | |
| | | | | | | | 25.8 | | | | | | |

### Example 3. Testing of the PolIII promoters in gene silencing constructs against a GUS reporter gene (Arabidopsis thaliana).

Similar constructs as in Example 2 were generated and cloned in pWBVec4a (see Table 4) and were used to transform a transgenic Arabidopsis line, expressing a CaMV35S-GUS gene.

**Table 4. Summary of constructs tested in Arabidopsis**

| **Constructs** | **Description** |
|---|---|
| pMBW479 | GUShp94 driven by 35S promoter (pART7) |
| pMBW480 | GUShp94 driven by AtU3+136 |
| pMBW481 | GUShp94 driven by AtU3 |
| pMBW482 | GUShp94 driven by At7SL (for reference only) |
| pMBW483 | GUShp94 driven by At7SL+86 (for reference only) |
| pMBW485 | GUShp94 driven by OsU3 |
| pMBW486 | GUShp94 driven by AtU3+3 |
| pMBW488 | GUShp94 driven by AtU3+20 |
| pLMW62 | GUShp94 driven by TomU3 |
| pLMW56 | GUShp41 driven by 35S promoter |
| pLMW52 | GUShp41 driven by AtU3+136 |
| PLMW60 | GUShp21 driven by AtU3+136 |

Leaf tissues from T1 plants that showed high-levels of resistance to the selective agent PPT were assayed for GUS activity. The MUG assay data are summarized in Table 5.

For the GUShp94 sequence all the U3 and U6 promoter-driven constructs conferred GUS silencing, although the TomU3 and AtU6+20 gave more consistent and better silencing.. The two At7SL promoter constructs did not appear to confer significant GUS silencing although a few lines showed moderate silencing, which may be due to T-DNA insertion next to endogenous promoters.

With the GUShp41 sequence, the AtU3+136 construct performed better than the 35S construct in terms of the degree of GUS silencing, again suggesting that PolIII promoters are more effective than PolII promoters for driving expression of small hairpin RNA expression in plants.

**Table 5. MUG assay of Arabidopsis leaf tissue super-transformed with constructs listed in Table 4 (5 µg protein)**

| Cons tructs | Untr ansformed | 479 | 480 | 481 | 482 | 483 | 485 | 486 | 488 | 62 | 56 | 52 | 60 |
|---|---|---|---|---|---|---|---|---|---|---|---|---|---|
| 35S-GUS back grou nd | 56.1 | 1.53 | 19.9 | 3.11 | 14.1 | 4.25 | 0.30 | 4.50 | 2.19 | 9.58 | 9.30 | 3.63 | 6.43 |
| | 65.5 | 0.33 | 3.50 | 2.37 | 8.53 | 75.3 | 6.79 | 14.4 | 1.99 | 0 | 6.98 | 1.52 | 55.3 |
| | 69.5 | 75.4 | 15.0 | 14.1 | 6.71 | | 30.2 | 0 | 7.97 | 0 | 7.42 | 3.80 | 55.3 |
| | 45.0 | 8.73 | 2.8 | 25.3 | 35.6 | | 3.88 | | 17.8 | 0 | 9.35 | | 63.1 |
| | 68.4 | 1.50 | 7.0 | 2.90 | 15.6 | | 16.4 | | 10.7 | 15.5 | 6.60 | | 8.07 |
| | | 1.13 | 18.9 | 4.53 | 48.3 | | 9.03 | | 1.08 | | 56.6 | | 29.0 |
| | | | 2.97 | 1.79 | 39.9 | | | | 6.83 | | | | 81.6 |
| | | | 2.48 | | 61.9 | | | | 2.96 | | | | |
| | | | 13.2 | | 94.4 | | | | 47.9 | | | | |
| | | | | | | | | | 4.32 | | | | |
| | | | | | | | | | 2.49 | | | | |
| | | | | | | | | | 10.1 | | | | |

### Example 4. Testing of the PolIII promoters in gene silencing constructs against a GUS reporter gene (Oryza sativa).

The constructs pMBW479, pMBW481, pMBW485, pMBW486 and pLMW62 (see Table 4) were super-transformed into rice that expresses a Ubil-GUS-nos gene. GUS staining showed that only pMBW485 (OsU3-GUShp94) and pMBW479 (35S-GUShp94) conferred significant silencing to the resident GUS gene. These results indicate that dicotyldonous type 3 PolIII promoters will not function in monocots.

### Example 5. Testing of the PolIII promoters in gene silencing constructs against Arabidopsis endogenous genes.

The Arabidopsis U6-26 construct contains the promoter from -446 to +3 bp (SEQ ID 5) and additional sequences added by PCR creating XhoI sites at each end of the fragment. These were used to clone the PCR product into the SalI site of a pGEM derived plasmid. The insert was excised with NotI and inserted into the pART27 binary vector for plant transformation. The PCR also incorporated a SalI site between the promoter and termination sequences (T8) for insertion of oligonucleotide sequences.

Two genes were targeted, phytoene desaturase (PDS - silencing gives a photobleached phenotype) and phytochrome B (PHYB - silencing gives hypocotyl elongation in white light). For PDS a single target region was chosen, for PHYB, three target regions were used, respectively from the 5'UTR, a region of the coding region conserved between phytochromes and the 3' UTR. For each target region two oligonucleotides were made, one to make a double stranded section of 21 bp long, the other to make a 42bp double stranded section. The double stranded oligos were made as two single strands (upper and lower) and annealed to form a double stranded DNA fragment. Overhang sequences were included at the 5' and 3' ends to create SalI compatible ends. The oligo sequences are represented in the sequence listing as SEQ ID 12 to 23 for the PHYB constructs and SEQ ID 24 to 27 for PDS constructs.

The PDShp42 constructs gave phenotypes in most of the examined plants. The results are summarized in table 6.

**Table 6. PDS scores (number of T1 seedlings showing phenotype)**

| **Phenotype** | **U6+PDS42** | **35S+PDS42** |
|---|---|---|
| No phenotype | 2 | 17 |
| Bleached cotyledons only | 0 | 26 |
| Total bleaching (cotyledons and 1^{st} pair of leafs | 43 | 0 |

Insertion of the construct with the dsRNA coding region PDS42 under control of the 35S promoter resulted in more plants with no silencing phenotype than the construct with the dsRNA coding region PDS42 under control of the U6 promoter, and plants with a phenotype only showed the weak bleached cotyledon phenotype and no bleaching of the leaves.

For the PHYB silencing experiments, most satisfactory silencing results are obtained with the PHYBC42 dsRNA coding region, where most of the plants show more elongation than the controls. Most of the other constructs do not show a phenotype or else only have one or two plants showing a phenotype suggesting that the choice of target sequence may be important.

Hypocotyl lengths of white light grown plants were measured and grouped in 5 mm categories.From the summary of the data in Table 7, it appears that the U6 promoter driven construct is more effective that the CaMV35S promoter driven construct, buth the results are not as pronounced as for the above mentioned PDS gene silencing experiments.

**Table 7. Silencing of PHYB.**

| Categories Hypocotyl length (cm) | WT | 35S-PHYbC42 | U6-PHYbC42 |
|---|---|---|---|
| 0.1-0.5 | | | |
| 0.6-1.0 | 13 | 1 | 2 |
| 1.1-1.5 | 10 | 2 | 5 |
| 1.6-2.0 | 3 | 5 | 10 |
| 2.1-2.5 | | 6 | 6 |
| 2.6-3.0 | 5 | | 9 |
| 3.1-3.5 | 3 | 3 | 2 |
| 3.6-4.0 | | 1 | 11 |
| 4.1-4.5 | | | 2 |
| 4.6-5.0 | | 1 | 1 |

Thus, the following conclusion scan be drawn from the experiments :
1. Type 3 Pol III promoters can be used to effectively drive the expression of dsRNA molecules in plant cells.
2. The At U6 promoter seems to be the most effective promoter tested.
3. The monocot PolIIII promoter is functional both in monocotyledonous and dicotyledonous plants, but the dicotyledonous promoters seem not to be functional in monocotyledonous plants.
4. The type III Pol III promoters appear to be more effective than CaMV35S promoter for gene silencing with relatively short hairpin sequences.

### Example 6. Additional experiments with small hairpin RNA encoding constructs

By using a the cloning strategy as outlined Figure 1, 20 new small hairpin constructs, as summarized in Table 7, were prepared. The predicted small hpRNAs from all of these constructs comprise a 42 bp dsRNA stem (corresponding to the target gene sequences) and a 9-nt loop (non-target sequence). Three target sequences corresponding to different regions of EIN2 (represented in SEQ ID 31 to 33) or GUS (represented in SEQ ID 28 to 30) were selected. These constructs have been used to transform tobacco to assess their efficacy for inducing the silencing of corresponding endogenous (EIN2) or reporter (GUS) genes.

Tobacco shoots were assayed for GUS expression, and the result is shown in **Table 8.**

The result shows that
1) most of the small GUS hairpin constructs conferred good GUS silencing; and
2) the PolIII promoter driven constructs pLMW164 and pLMW165 result in more consistent GUS silencing than the 35S promoter driven construct pLMW155.

**Table 7. Summary of additional small hairpinRNA encoding constructs**

| **Name** | **Promoter** | **Hairpin sequence (42 nt stem)** | **Name** | **Promoter** | **Hairpin sequence** |
|---|---|---|---|---|---|
| **pLMW154** | 35S | GUS-A (SEQ ID No 28) | **pLMW164** | TomU3 | GUS-A (SEQ ID No 28) |
| **pLMW155** | 35S | GUS-B(SEQ ID No 29) | **pLMW165** | TomU3 | GUS-B (SEQ ID No 29) |
| **pLMW156** | 35S | GUS-C(SEQ ID No 30) | **pLMW166** | TomU3 | GUS-C (SEQ ID No 30) |
| **pLMW157** | 35S | EIN-A(SEQ ID No 31) | **pLMW167** | TomU3 | EIN-A (SEQ ID No 31) |
| **pLMW158** | 35S | EIN-B(SEQ ID No 32) | **pLMW168** | TomU3 | EIN-B (SEQ ID No 32) |
| **pLMW159** | AtU3B | GUS-A(SEQ ID No 28) | **pLMW169** | AtU6 | GUS-A (SEQ ID No 28) |
| **pLMW160** | AtU3B | GUS-B(SEQ ID No 29) | **pLMW170** | AtU6 | GUS-B (SEQ ID No 29) |
| **pLMW161** | AtU3B | GUS-C(SEQ ID No 30) | **pLMW171** | AtU6 | GUS-C (SEQ ID No 30) |
| **pLMW162** | AtU3B | EIN-A(SEQ ID No 31) | **pLMW172** | AtU6 | EIN-A (SEQ ID No 31) |
| **pLMW163** | AtU3B | EIN-B(SEQ ID No 32) | **pLMW173** | AtU6 | EIN-B (SEQ ID No 32) |

**Table 8: GUS activity of putatively transformed tobacco shoots**

| Constru ct | Untransformed | LMW 155 | LMW 156 | LMW 164 | LMW 165 | LMW 166 | LMW 169 | LMW 170 |
|---|---|---|---|---|---|---|---|---|
| MUG assay reading | 51.2 | 7.14 | 0.76 | 2.16 | 12.4 | 1.27 | 0.94 | 1.75 |
| | 37.0 | 20.2 | | 0.40 | 1.55 | 68.3 | 1.45 | 2.40 |
| | 52.2 | 0.16 | | 1.41 | 2.22 | 27.6 | 1.83 | 25.32 |
| | 44.32 | 8.98 | | | 0.81 | 7.07 | 7.74 | 0.70 |
| | | 3.04 | | | 2.31 | | 37.5 | 52.7 |
| | | 0.17 | | | 1.19 | | 3.35 | 26.0 |
| | | 70.3 | | | 1.24 | | 25.3 | 7.65 |
| | | 74.3 | | | | | 85.3 | 1.31 |
| | | 81.9 | | | | | | 2.00 |
| | | | | | | | | 9.18 |
| | | | | | | | | 0.69 |
| | | | | | | | | 19.6 |
| | | | | | | | | 1.37 |
| | | | | | | | | 1.75 |
| | | | | | | | | 0.92 |
| | | | | | | | | 35.3 |

The tobacco tissue assayed was mostly leaf pieces from small regenerating shoots growing on medium with hygromycin, which usually gives tight selection.

### References

Bourque and Folk (1992) Plant Mol. Biol.19: 641-647
Brummelkamp et al. (2002) Science 296 : 550-553
Elbashir et al. (2001) Nature 411: 494-498
Fire et al. (1998) Nature 391: 806-811
Hamilton et al. (1998) Plant J. 15: 737-746
Miyagishi et al. (2002) Nature Biotechnology 20: 497-499
Needleman and Wunsch (1970) J. Mol. Biol. 48: 443-453
Paddison et al. (2002) Genes & Development 16: 948-958
Paul et al. (2002) Nature Biotechnology 20: 505-508
Sook Lee et al. (2002) Nature Biotechnology 20: 500-505
Sui et al. (2002) Proc. Natl. Acad. Sci. USA 99: 5515-5520
Waterhouse et al (1998) Proc. Natl. Acad. Sci. USA 95: 13959-13964
Yukawa et al. (2002) Plant Mol. Biol. 50: 713-723

## Claims

1. A method for reducing the expression of a gene of interest in a plant cell, comprising the following steps:
(a) providing a chimeric gene to said plant cell, said chimeric gene comprising the following operably linked DNA fragments :
i) a promoter recognized by a DNA dependent RNA polymerase III of said plant cell which is a promoter of type 3;
ii) a DNA fragment which, when transcribed, yields an RNA molecule, said RNA molecule comprising a sense and antisense nucleotide sequence,
(1) said sense nucleotide sequence comprising about 19 contiguous nucleotides having about 90 to about 100% sequence identity to a nucleotide sequence of about 19 contiguous nucleotide sequences from the RNA transcribed from said gene of interest;
(2) said antisense nucleotide sequence comprising about 19 contiguous nucleotides having about 90 to 100% sequence identity to the complement of a nucleotide sequence of about 19 contiguous nucleotide sequence of said sense sequence;
wherein said sense and antisense nucleotide sequence are capable of
forming a double stranded RNA consisting of about 19 to about 200 nucleotides in length ; and
iii) an oligo dT stretch comprising at least 4 consecutive T-residues;
wherein said chimeric gene is **characterized in that** said chimeric gene has all its cis-acting elements which interact with said DNA dependent RNA polymerase III upstream of the region transcribed by RNA polymerase III; and
(b) identifying plant cells wherein said expression of said gene of interest is reduced when compared to the expression of said gene of interest in plant cells which do not comprise said chimeric gene.

2. The method according to claim 1, wherein said promoter is a type 3 POLIII promoters selected from the promoter of a plant gene encoding U6snRNA or the promoter of a plant gene encoding U3snRNA.

3. The method according to claim 1 or claim 2, wherein said promoter comprises a nucleotide sequence selected from the nucleotide sequences of SEQ ID Nr 3 from the nucleotide at position 7 to the nucleotide at position 313, SEQ ID Nr 4 from the nucleotide at position 7 to the nucleotide at position 446, SEQ ID Nr 5 from the nucleotide at position 7 to the nucleotide at position 436, SEQ ID Nr 6 from the nucleotide at position 7 to the nucleotide at position 468, SEQ ID Nr 7 from the nucleotide at position 7 to the nucleotide at position 384 or SEQ ID Nr 8 from the nucleotide at position 7 to the nucleotide at position 421.

4. The method according to any one of claims 1 to 3, wherein said plant cell is a dicotyledonous plant cell and said promoter is derived from a dicotyledonous or monocotyledonous plant or plant cell.

5. The method according to any one of claims 1 to 3, wherein said plant cell is a monocotyledonous plant cell, and said promoter is derived from a monocotyledonous plant or plant cell.

6. The method according to any one of claims 1 to 3 wherein said promoter is endogenous to said plant cell.

7. The method according to any one of claims 1 to 6, wherein said gene of interest is a transgene.

8. The method according to any one of claims 1 to 6, wherein said gene of interest is an endogenous gene.

9. The method according to any one of claims 1 to 8, wherein said plant cell is comprised within a plant.

10. A chimeric gene as described in any of claims 1 to 9.

11. A plant cell comprising a chimeric gene according to claim 10.

12. A plant comprising within its plant cells a chimeric gene according to claim 10.

## Patentansprüche

1. Verfahren zum Verringern der Expression eines Gens von Interesse in einer Pflanzenzelle, umfassend die nachstehend aufgeführten Schritte:
(a) der Pflanzenzelle ein chimäres Gen bereitstellen, wobei das chimäre Gen die nachstehend aufgeführten, wirksam verbundenen DNA Fragmente umfasst:
i) einen Promotor, welcher durch eine DNA abhängige RNA Polymerase III der Pflanzenzelle erkannt wird, worin der Promotor vom Typ 3 ist;
ii) ein DNA Fragment, welches bei Transkription ein RNA Molekül erzeugt, worin das RNA Molekül eine Sense und Antisense Nukleotidsequenz umfasst,
(1) worin die Sense Nukleotidsequenz ungefähr 19 kontinuierliche Nukleotide mit ungefähr 90 bis ungefähr 100% Sequenzidentität zu einer Nukleotidsequenz von ungefähr 19 kontinuierlichen Nukleotidsequenzen von der RNA umfasst, welche von dem Gen von Interesse transkribiert wird;
(2) worin die Antisense Nukleotidsequenz ungefähr 19 kontinuierliche Nukleotide mit ungefähr 90 bis ungefähr 100% Sequenzidentität zu dem Komplement einer Nukleotidsequenz von ungefähr 19 kontinuierlichen Nukleotidsequenzen der Sense Sequenz umfasst;
worin die Sense und Antisense Nukleotidsequenz eine doppelsträngige RNA bilden können, welche ein Länge von ungefähr 19 bis ungefähr 200 Nukleotiden umfasst; und
iii) ein Oligo dT Strang umfassend mindestens 4 aufeinanderfolgende T-Reste;
worin das chimäre Gen **dadurch gekennzeichnet ist, dass** das chimäre Gen alle seine Ciswirkenden Elemente, welche mit der DNA abhängigen RNA Polymerase III wechselwirken, oberhalb des durch RNA Polymerase III transkribierten Bereich aufweist und
(b) Identifizieren von Pflanzenzellen, worin die Expression des Gens von Interesse verringert ist, sofern mit der Expression des Gens von Interesse in Pflanzenzellen verglichen wird, die das chimäre Gen nicht enthalten.

2. Verfahren nach Anspruch 1, worin der Promotor ein Typ 3 POLIII Promotor ist, ausgewählt unter dem Promotor eines U6snRNA kodierenden Pflanzengens oder dem Promotor eines U3snRNA kodierenden Pflanzengens.

3. Verfahren nach Anspruch 1 oder Anspruch 2, worin der Promotor eine Nukleotidsequenz umfasst, ausgewählt unter den Nukleotidsequenzen der SEQ ID Nr. 3 von dem Nukleotid an Position 7 bis zu dem Nukleotid an Position 313, SEQ ID Nr. 4 von dem Nukleotid an Position 7 bis zu dem Nukleotid an Position 446, SEQ ID Nr. 5 von dem Nukleotid an Position 7 bis zu dem Nukleotid an Position 436, SEQ ID Nr. 6 von dem Nukleotid an Position 7 bis zu dem Nukleotid an Position 468, SEQ ID Nr. 7 von dem Nukleotid an Position 7 bis zu dem Nukleotid an Position 384, oder SEQ ID Nr. 8 von dem Nukleotid an Position 7 bis zu dem Nukleotid an Position 421.

4. Verfahren nach einem der Ansprüche 1 bis 3, worin die Pflanzenzelle eine zweikeimblättrige Pflanzenzelle ist und der Promotor von einer zweikeimblättrigen oder einkeimblättrigen Pflanze oder Pflanzenzelle abgeleitet ist.

5. Verfahren nach einem der Ansprüche 1 bis 3, worin die Pflanzenzelle eine einkeimblättrige Pflanzenzelle ist und der Promotor von einer einkeimblättrigen Pflanze oder Pflanzenzelle abgeleitet ist.

6. Verfahren nach einem der Ansprüche 1 bis 3, worin der Promotor für die Pflanze endogen ist.

7. Verfahren nach einem der Ansprüche 1 bis 6, worin das Gen von Interesse ein Transgen ist.

8. Verfahren nach einem der Ansprüche 1 bis 6, worin das Gen von Interesse ein endogenes Gen ist.

9. Verfahren nach einem der Ansprüche 1 bis 8, worin die Pflanzenzelle in einer Pflanze beinhaltet ist.

10. Chimäres Gen, wie in einem der Ansprüche 1 bis 9 beschrieben.

11. Pflanzenzelle umfassend ein chimäres Gen nach Anspruch 10.

12. Pflanze umfassend in seinen Pflanzenzellen ein chimäres Gen nach Anspruch 10.

## Revendications

1. Procédé pour réduire l'expression d'un gène d'intérêt dans une cellule végétale, comprenant les étapes suivantes :
(a) la fourniture à ladite cellule végétale d'un gène chimère, ledit gène chimère comprenant les fragments d'ADN suivants liés de manière fonctionnelle :
i) un promoteur reconnu par une ARN polymérase III dépendante de l'ADN de ladite cellule végétale qui est un promoteur de type 3 ;
ii) un fragment d'ADN qui, lorsqu'il est transcrit, produit une molécule d'ARN, ladite molécule d'ARN comprenant une séquence nucléotidique sens et antisens,
(1) ladite séquence nucléotidique sens comprenant environ 19 nucléotides contigus ayant environ 90 à environ 100% d'identité de séquence à une séquence nucléotidique d'environ 19 séquences nucléotidiques contigües de l'ARN transcrit à partir dudit gène d'intérêt ;
(2) ladite séquence nucléotidique antisens comprenant environ 19 nucléotides contigus ayant environ 90 à 100% d'identité de séquence au complément d'une séquence nucléotidique d'environ 19 séquence nucléotidique contiguë de ladite séquence sens ;
dans lequel lesdites séquences nucléotidiques sens et antisens sont capables de former un ARN double brin constitué d'environ 19 à environ 200 nucléotides de long ; et
iii) une étendue oligo dT comprenant au moins 4 résidus T consécutifs ;
dans lequel ledit gène chimère est **caractérisé en ce que** ledit gène chimère a tous ses éléments agissant en cis qui interagissent avec ladite ARN polymérase III dépendante de l'ADN en amont de la région transcrite par l'ARN polymérase III ; et
(b) l'identification des cellules végétales dans lesquelles ladite expression dudit gène d'intérêt est réduite par rapport à l'expression dudit gène d'intérêt dans des cellules végétales qui ne comprennent pas ledit gène chimère.

2. Procédé selon la revendication 1, dans lequel ledit promoteur est un promoteur POLIII de type 3 choisi parmi le promoteur d'un gène végétal codant pour U6snRNA ou le promoteur d'un gène végétal codant pour U3snRNA.

3. Procédé selon la revendication 1 ou la revendication 2, dans lequel ledit promoteur comprend une séquence nucléotidique choisie parmi les séquences nucléotidiques de SEQ ID No 3 du nucléotide en position 7 au nucléotide en position 313, SEQ ID No 4 du nucléotide en position 7 au nucléotide en position 446, SEQ ID No 5 du nucléotide en position 7 au nucléotide en position 436, SEQ ID No 6 du nucléotide en position 7 au nucléotide en position 468, SEQ ID No 7 du nucléotide en position 7 au nucléotide en position 384 ou SEQ ID No 8 du nucléotide en position 7 au nucléotide en position 421.

4. Procédé selon l'une quelconque des revendications 1 à 3, dans lequel ladite cellule végétale est une cellule de plante dicotylédone et ledit promoteur est dérivé d'une plante ou d'une cellule de plante dicotylédone ou monocotylédone.

5. Procédé selon l'une quelconque des revendications 1 à 3, dans lequel ladite cellule végétale est une cellule de plante monocotylédone, et ledit promoteur est dérivé d'une plante ou d'une cellule de plante monocotylédone.

6. Procédé selon l'une quelconque des revendications 1 à 3, dans lequel ledit promoteur est endogène à ladite cellule végétale.

7. Procédé selon l'une quelconque des revendications 1 à 6, dans lequel ledit gène d'intérêt est un transgène.

8. Procédé selon l'une quelconque des revendications 1 à 6, dans lequel ledit gène d'intérêt est un gène endogène.

9. Procédé selon l'une quelconque des revendications 1 à 8, dans lequel ladite cellule végétale est comprise dans une plante.

10. Gène chimère tel que décrit dans l'une quelconque des revendications 1 à 9.

11. Cellule végétale comprenant un gène chimère selon la revendication 10.

12. Plante comprenant dans ses cellules végétales un gène chimère selon la revendication 10.
